# EUROPÄISCHE PATENTSCHRIFT

(11) **EP 0 489 053 B1**
(45) Veröffentlichungstag und Bekanntmachung des Hinweises auf die Patenterteilung: **07.09.1994**
(21) Anmeldenummer: 90912361.4
(22) Anmeldetag: 18.08.1990
(51) Int. Cl.: C07D 251/28, C07B 63/00

(54) **VERFAHREN ZUR VERNICHTUNG VON CYANURFLUORID IN DEN BEI DESSEN HERSTELLUNG ANFALLENDEN RÜCKSTÄNDEN**
PROCESS FOR DESTROYING CYANURIC FLUORIDE IN RESIDUES PRODUCED IN ITS MANUFACTURE
PROCEDE POUR DETRUIRE LE FLUORURE DE CYANURE DANS LES RESIDUS PRODUITS LORS DE SA FABRICATION

(30) Priorität: 24.08.1989 DE 3927951
(43) Veröffentlichungstag der Anmeldung: 10.06.1992
(73) Patentinhaber: HOECHST AKTIENGESELLSCHAFT, 65926 Frankfurt am Main (DE)
(72) Erfinder: GRÖTSCH, Georg, D-6238 Hofheim am Taunus (DE)
(86) Internationale Anmeldenummer: EP9001363
(87) Internationale Veröffentlichungsnummer: WO9102727

(56) Entgegenhaltungen:
- EP-A- 0 035 704
- GB-A- 2 194 238

## Beschreibung

Die Erfindung betrifft ein Verfahren zur sicheren und quantitativen Vernichtung von Cyanurfluorid in den bei dessen Herstellung aus Cyanurchlorid und Alkalimetallfluoriden in dipolar aprotischen Solventien anfallenden Rückständen.

Es ist seit längerem bekannt, daß man Cyanurfluorid aus Cyanurchlorid mit Alkalimetallfluoriden, wie z.B. NaF (Tullock, Coffman, J. Org. Chem. 25, 2016 (1960), EP-PS 035 704) oder KF oder CsF (JP 61 047 465) in dipolar aprotischen Solventien, wie z.B. Sulfolan oder Benzonitril unter wasserfreien Bedingungen herstellen kann. Cyanurfluorid wird hierbei durch Destillation bei Normaldruck oder im Vakuum, gegebenenfalls in Gegenwart eines Schleppmittels, aus der Reaktionsmischung entfernt. Es dient als wertvolles Vorprodukt für Agrochemikalien, Farbstoffe, optische Aufheller, Photochemikalien und Pharmazeutika.

Die technische Nutzung dieser Verfahren hat den großen Nachteil, daß im Destillationsrückstand neben dem gebildeten Alkalimetallchlorid normalerweise Spuren an Cyanurfluorid verbleiben bzw. daß bei der Destillation des Cyanurfluorids ein Zwischenlauf anfällt, der Cyanurfluorid enthält. Aufgrund der hohen Toxizität von Cyanurfluorid (LC₅₀ (Inhal-Ratte): 3,1 ppm; LD₅₀ (Haut-Kaninchen): 160 ppm, J. Am. Ind. Hyg. Ass. 33, 382 (1972)) müssen daher diese Rückstände vor einer endgültigen Entsorgung entgiftet werden. Eine ggf. durchgeführte Aufarbeitung unter Wiederverwendung des Solvens, z.B. Sulfolan, ist nach Vernichtung des Cyanurfluorids ebenfalls bedeutend einfacher und kostengünstiger, da die für den Umgang mit Cyanurfluorid benötigten sicherheitstechnischen Maßnahmen entfallen können.

Es bestand somit ein Bedarf für ein Verfahren, bei welchem nicht nur wie nach DE-OS 37 27 973 (=GB-A 2 194 238) u.U. vorhandene Anteile von HF gebunden werden, sondern zusätzlich des noch verbliebene, restliche Cyanurfluorid im Destillationsrückstand bzw. in einem Destillationszwischenlauf sicher vernichtet werden kann. Eine Behandlung der Destillationsrückstände mit Kalkmilch (Kühn-Birett, Merkblätter Gefährliche Arbeitsstoffe, Blatt-Nr. C 73) ist für die technische Durchführung ungeeignet, da der Reaktionsbehälter nach einer Verunreinigung mit Wasser nur nach gründlicher Reinigung - d.h. vor allem Trocknung - erneut für die Cyanurfluorid-Herstellung eingesetzt werden kann. Das Lösungsmittel muß vor dem Wiedereinsatz ebenfalls aufwendig getrocknet werden.

Es wurde nun gefunden, daß das in Rückständen aus der Cyanurfluorid-Herstellung durch Umsetzung von Cyanurchlorid mit Alkalimetallfluoriden in dipolar aprotischen Lösungsmitteln nach destillativer Abtrennung der Hauptmenge an Cyanurfluorid noch verbliebene restliche Cyanurfluorid sicher und quantitativ vernichtet werden kann, indem man den cyanurfluoridhaltigen Rückstand mit der zum restlichen Cyanurfluorid mindestens äquivalenten Menge an bis auf ggfs. vorhandenes Kristallwasser wasserfreiem Alkalimetallhydroxid, -hydrogencarbonat oder -carbonat oder einem beliebigen Gemisch dieser Verbindungen bei Temperaturen von etwa 20 bis etwa 180°C, vorzugsweise von etwa 50 bis etwa 140°C, besonders bevorzugt von etwa 70 bis etwa 130°C umsetzt.

Als Alkalimetallhydroxid, -hydrogencarbonat oder -carbonat kommen beispielsweise LiOH, NaOH, KOH, NaHCO₃, KHCO₃, Na₂CO₃ oder K₂CO₃ oder Mischungen daraus in Frage.

Die Anwendung von Alkalimetallhydroxid, -hydrogencarbonat oder -carbonat in mindestens äquivalenter Menge, bezogen auf das zu vernichtende restliche Cyanurfluorid, bedeutet die Anwendung von mindestens 3 mol Alkalimetallhydroxid bzw. -hydrogencarbonat, und von mindestens 1,5 mol Alkalimetallcarbonat pro mol Cyanurfluorid. Es ist jedoch zweckmäßig, einen über die äquivalente Menge hinausgehenden Überschuß von etwa 10 bis etwa 300 mol%, vorzugsweise von etwa 50 bis etwa 250 mol% an den genannten Alkalimetallverbindungen einzusetzen.

Wendet man Mischungen aus Alkalimetallhydroxiden, -hydrogencarbonaten und -carbonaten an, so werden die genannten Komponenten, ihrem Anteil in der Mischung entsprechend, in den vorstehend genannten Mengen pro mol Cyanurfluorid eingesetzt.

Der Zusatz der genannten Alkalimetallverbindungen zu den cyanurfluoridhaltigen Rückständen erfolgt entweder in Substanz oder in Form einer Suspension der Alkalimetallverbindung vorzugsweise in dem als Reaktionsmedium benutzten dipolar aprotischen Lösungsmittel, wie beispielsweise Sulfolan, Nitrobenzol oder Benzonitril, oder in einem anderen, unter den Reaktionsbedingungen inerten Lösungsmittel, das sich bevorzugterweise destillativ von dem als Reaktionsmedium verwendeten dipolar aprotischen Lösungsmittel abtrennen läßt. Als solche inerte Lösungsmittel kommen aprotische Lösungsmittel, wie beispielsweise Toluol, Chlorbenzol oder Dichlorbenzol, in Frage.

Der Zusatz der genannten Alkalimetallverbindungen in Substanz oder in Form einer Suspension in einem aprotischen Lösungsmittel zum cyanurfluoridhaltigen Rückstand erfolgt zweckmäßigerweise fortlaufend in Anteilen, d.h. durch Zudosieren.

Wie die gaschromatographische Kontrolle der Umsetzung zeigt, ist nach kurzer Zeit Cyanurfluorid nicht mehr nachweisbar, d.h. auch Spuren an Cyanurfluorid werden sicher vernichtet.

Da keine wäßrigen Basen eingesetzt werden, wird die Verunreinigung des Lösungsmittels und der Reaktionsapparatur mit Wasser vermieden.

Die Wiedergewinnung der jeweils eingesetzten Lösungsmittel kann durch Filtration oder durch Destillation bei Normaldruck oder im Vakuum erfolgen. Die auf diese Weise nahezu quantitativ zurückerhaltenen Lösungsmittel können erneut verfahrensgemäß oder zur Herstellung von Cyanurfluorid eingesetzt werden. Der nach Abtrennung des Lösungsmittels erhaltene Rückstand an cyanurfluoridfreiem Alkalimetallchlorid, gemischt mit überschüssigem Alkalimetallfluorid, z.B. NaCl/NaF, KCl/KF, CsCl/CsF oder ein beliebiges Gemisch dieser Alkalimetallsalze, kann anschließend problemlos gelagert bzw. aufgearbeitet werden.

Überraschenderweise sind die oft ebenfalls als Basen einsetzbaren Verbindungen Calciumhydroxid, Calciumoxid, Calciumcarbonat, Magnesiumoxid oder Magnesiumhydroxid weitaus weniger geeignet, Cyanurfluorid schnell und quantitativ abzubauen (siehe die Vergleichsbeispiele 7 bis 11).

Darüberhinaus war nicht vorauszusehen, daß Basen als Feststoffe, wie Alkalimetallhydroxide, -carbonate oder -hydrogencarbonate Cyanurfluorid in geeigneter Weise zersetzen, ohne daß Wasser zugegen ist, das bekanntermaßen eine äußerst rasche Hydrolyse von Cyanurfluorid bewirkt (Seel et al., Chem. Ber. 92, 344 (1959)).

Die nachstehenden Beispiele dienen zur Erläuterung der Erfindung, ohne sie darauf zu beschränken.

### Beispiel 1

Eine Mischung aus 21,0 g Sulfolan und 1,1 g Cyanurfluorid wird mit 2,1 g NaOH-Plätzchen bei Raumtemperatur versetzt. Nach zweistündigem Rühren ist kein Cyanurfluorid mehr nachweisbar.

### Beispiel 2

Eine Mischung aus 68,9 g Sulfolan und 7,7 g Cyanurfluorid wird bei etwa 25°C mit 6,8 g NaOH-Pulver versetzt. Unter schnellem Temperaturanstieg auf etwa 180°C reagiert Cyanurfluorid quantitativ mit der Base ab (GC-Kontrolle).

### Beispiel 3

Eine Mischung aus 68,9 g Sulfolan und 7,7 g Cyanurfluorid wird bei Raumtemperatur mit 19,8 g Na₂CO₃ versetzt. Bei etwa 25 bis 40°C reagiert Cyanurfluorid in 1,5 h quantitativ mit der Base unter CO₂-Entwicklung ab (GC-Kontrolle).

### Beispiel 4

Wie in Beispiel 3 beschrieben werden 7,7 g Cyanurfluorid mit 28,6 g NaHCO₃ zur Reaktion gebracht. Nach 1,5 h ist Cyanurfluorid nicht mehr mit GC nachweisbar.

### Beispiel 5

Zu einer Suspension von 384 g Kaliumfluorid in 700 g Sulfolan wird bei 100°C eine ebenfalls 100°C heiße Lösung von 369 g Cyanurchlorid in 500 g Sulfolan innerhalb von 2 h zugetropft. Anschließend wird Cyanurfluorid bei einem Druck von 500 bis 100 mbar abdestilliert. Die Ausbeute beträgt 257 g (95 % d.Th.); Gehalt 99,4 Flächen-% (GC).

Der Destillationsrückstand enthält laut GC noch 1 Gew.-% Cyanurfluorid, bezogen auf Sulfolan. Nach Zugabe von 40 g Soda (calciniert) wird noch 1 h bei dieser Temperatur nachgerührt. Cyanurfluorid ist dann mit GC nicht mehr nachweisbar.

Nach Abfiltrieren der ungelösten Salze bei 60°C wird die Sulfolan-Mutterlauge bei 27 bar/160°C destilliert. Man erhält 1128 g Sulfolan. Zusammen mit dem Trocknungskondensat des Kaliumchlorid/-fluorid-Gemisches werden 98 % des eingesetzten Sulfolans zurückgewonnen.

### Beispiel 6

Ein wie in Beispiel 5 erhaltener Destillationssumpf wird bei 100°C mit 40 g Pottasche versetzt und 1 h bei dieser Temperatur gerührt; Cyanurfluorid ist mit GC anschließend nicht mehr nachweisbar.

### Beispiel 7 (Vergleichsbeispiel)

Ein Gemisch aus 21,1 g Sulfolan und 1,0 g Cyanurfluorid wird mit 2,0 g Calciumhydroxid versetzt und 1 h bei Raumtemperatur gerührt. Gemäß GC hat sich der Cyanurfluorid-Gehalt von 4,5 Gew.-% auf 4,0 Gew.-%, bezogen auf die flüssige Phase, vermindert. Erst nach anschließendem 2-stündigem Heizen auf 85°C ist der Cyanurfluorid-Gehalt auf 1,9 %, bezogen auf Sulfolan, gefallen.

### Beispiel 8 (Vergleichsbeispiel)

Ein Gemisch aus 21,6 g Sulfolan und 1,1 g Cyanurfluorid wird mit 2,13 g Magnesiumoxid versetzt. Es wird 1 h bei Raumtemperatur, anschließend 7,5 h bei 85°C gerührt. Der Cyanurfluoridanteil ist nach dieser Zeit von ursprünglich 4,8 Gew.-% auf 1,2 Gew.-%, bezogen auf die flüssige Phase, gesunken.

### Beispiel 9 (Vergleichsbeispiel)

Eine Mischung aus 68,9 g Sulfolan und 7,7 g Cyanurfluorid wird mit 10,8 g Magnesiumhydroxid versetzt und 3,5 h bei Raumtemperatur sowie anschließend 2 h bei 100°C gerührt. Der Cyanurfluoridanteil ist von ursprünglich 10 Gew.-% auf 8 Gew.-%, bezogen auf die flüssige Phase, gesunken.

### Beispiele 10 und 11 (Vergleichsbeispiele)

Eine Mischung aus 68,9 g Sulfolan/7,7 g Cyanurfluorid wird mit der angegebenen Base versetzt und zunächst bei Raumtemperatur, dann bei 100°C gerührt.

| Bsp. | Base | Menge (g) | Reaktionszeit Raumtemp. (h) | bei 100°C (h) | Anfangs-Cyanurfluorid, flüssige Phase (Gew.-%) | Endkonzentration bezogen auf die (Gew.-%) |
|---|---|---|---|---|---|---|
| 10 | CaO | 10,4 | 3,75 | 1,5 | 10 | 4,0 |
| 11 | CaCO₃ | 18,6 | 3,0 | 1,5 | 10 | 7,6 |

## Patentansprüche

1. Verfahren zur sicheren und quantitativen Vernichtung des in Rückständen aus der Cyanurfluorid-Herstellung durch Umsetzung von Cyanurchlorid mit Alkalimetallfluoriden in dipolar aprotischen Lösungsmitteln nach destillativer Abtrennung der Hauptmenge an Cyanurfluorid noch verbliebenen restlichen Cyanurfluorids, dadurch gekennzeichnet, daß man den Rückstand mit der zum restlichen Cyanurfluorid mindestens äquivalenten Menge an bis auf ggfs. vorhandenes Kristallwasser wasserfreiem Alkalimetallhydroxid, -hydrogencarbonat oder -carbonat oder einem beliebigen Gemisch dieser Verbindungen bei Temperaturen von 20 bis 180°C umsetzt.

2. Verfahren nach Anspruch 1, dadurch gekennzeichnet, daß man bei Temperaturen von 50 bis 140°C umsetzt.

3. Verfahren nach mindestens einem der Ansprüche 1 und 2, dadurch gekennzeichnet, daß man bei Temperaturen von 70 bis 130°C umsetzt.

4. Verfahren nach mindestens einem der Ansprüche 1 bis 3, dadurch gekennzeichnet, daß man den Rückstand mit mindestens 3 mol Alkalimetallhydroxid bzw. -hydrogencarbonat pro mol Cyanurfluorid umsetzt.

5. Verfahren nach mindestens einem der Ansprüche 1 bis 3, dadurch gekennzeichnet, daß man den Rückstand mit mindestens 1,5 mol Alkalimetallcarbonat pro mol Cyanurfluorid umsetzt.

6. Verfahren nach mindestens einem der Ansprüche 1 bis 3, dadurch gekennzeichnet, daß man bei Umsetzung des Rückstands mit einem Gemisch aus Alkalimetallhydroxid, -hydrogencarbonat und -carbonat diese Komponenten, ihrem Anteil in der Mischung entsprechend, in Mengen gemäß Ansprüchen 4 und 5 pro mol Cyanurfluorid einsetzt.

7. Verfahren nach mindestens einem der Ansprüche 1 bis 6, dadurch gekennzeichnet, daß man die Alkalimetallhydroxide, -hydrogencarbonate oder -carbonate oder deren Gemische dem cyanurfluoridhaltigen Rückstand in Substanz zusetzt.

8. Verfahren nach mindestens einem der Ansprüche 1 bis 6, dadurch gekennzeichnet, daß man die Alkalimetallhydroxide, -hydrogencarbonate oder -carbonate oder deren Gemische dem cyanurfluoridhaltigen Rückstand in Form einer Suspension in einem aprotischen Lösungsmittel, das sich destillativ von dem als Reaktionsmedium dienenden Lösungsmittel abtrennen läßt, zusetzt.

9. Verfahren nach mindestens einem der Ansprüche 1 bis 6 und 8, dadurch gekennzeichnet, daß man die Alkalimetallhydroxide, -hydrogencarbonate oder -carbonate oder deren Gemische dem cyanurfluoridhaltigen Rückstand, suspendiert in dem als Reaktionsmedium dienenden dipolar aprotischen Lösungsmittel, zusetzt.

10. Verfahren nach mindestens einem der Ansprüche 1 bis 6, dadurch gekennzeichnet, daß man den cyanurfluoridhaltigen Rückstand den Alkalimetallhydroxiden, -hydrogencarbonaten oder -carbonaten oder deren Gemischen zusetzt.

## Claims

1. A process for the reliable and quantitative destruction of the residual cyanuric fluoride which still remains in residues from the cyanuric fluoride preparation by reaction of cyanuric chloride with alkali metal fluorides in dipolar aprotic solvents after distillative removal of the majority of cyanuric fluoride, which comprises reacting the residue with an alkali metal hydroxide, bicarbonate or carbonate which is anhydrous apart from any water of crystallization present, or any desired mixture of these compounds, in an amount which is at least equivalent to the residual cyanuric fluoride at temperatures of 20 to 180°C.

2. The process as claimed in claim 1, wherein the reaction is carried out at temperatures of 50 to 140°C.

3. The process as claimed in at least one of claims 1 and 2, wherein the reaction is carried out at temperatures of 70 to 130°C.

4. The process as claimed in at least one of claims 1 to 3, wherein the residue is reacted with at least 3 mol of alkali metal hydroxide or bicarbonate per mol of cyanuric fluoride.

5. The process as claimed in at least one of claims 1 to 3, wherein the residue is reacted with at least 1.5 mol of alkali metal carbonate per mol of cyanuric fluoride.

6. The process as claimed in at least one of claims 1 to 3, wherein, in the reaction of the residue with a mixture of alkali metal hydroxide, bicarbonate and carbonate, these components are used in amounts as claimed in either of claims 4 and 5 per mol of cyanuric fluoride, according to their proportion in the mixture.

7. The process as claimed in at least one of claims 1 to 6, wherein the alkali metal hydroxide, bicarbonate or a carbonate or mixture thereof is added in bulk to the residue containing cyanuric fluoride.

8. The process as claimed in at least one of claims 1 to 6, wherein the alkali metal hydroxide, bicarbonate or carbonate or mixture thereof is added in the form of a suspension in an aprotic solvent, which can be separated off by distillation from the solvent used as the reaction medium, to the residue containing cyanuric fluoride.

9. The process as claimed in at least one of claims 1 to 6, wherein the alkali metal hydroxide, bicarbonate or carbonate or mixture thereof is added as a suspension in the dipolar aprotic solvent used as the reaction medium to the residue containing cyanuric fluoride.

10. The process as claimed in at least one of claims 1 to 6, wherein the residue containing cyanuric fluoride is added to the alkali metal hydroxide, bicarbonate or carbonate or mixture thereof.

## Revendications

1. Procédé pour détruire d'une manière sûre et quantitative le fluorure de cyanuryle résiduel encore restant, dans les résidus de la fabrication du fluorure de cyanuryle par réaction du chlorure de cyanuryle avec des fluorures de métaux alcalins dans des solvants aprotiques dipolaires par séparation par distillation de la quantité principale de fluorure de cyanuryle, caractérisé en ce qu'on fait réagir le résidu à des températures de 20 à 180°C avec la quantité au moins équivalente à celle du fluorure de cyanuryle résiduel, d'un hydroxyde, d'un hydrogénocarbonate ou d'un carbonate d'un métal alcalin anhydre jusqu'à l'eau de cristallisation éventuellement présente, ou à un mélange quelconque de ces composés.

2. Procédé selon la revendication 1, caractérisé en ce que la réaction est mise en oeuvre à des températures de 50 à 140°C.

3. Procédé selon au moins l'une des revendications 1 et 2, caractérisé en ce que la réaction est mise en oeuvre à des températures de 70 à 130°C.

4. Procédé selon au moins l'une des revendications 1 à 3, caractérisé en ce qu'on fait réagir le résidu avec au moins 3 moles d'hydroxyde ou d'hydrogénocarbonate d'hydroxyde d'un métal alcalin par mole de fluorure de cyanuryle.

5. Procédé selon au moins l'une des revendications 1 à 3, caractérisé en ce qu'on fait réagir le résidu avec au moins 1,5 moles de carbonate d'un métal alcalin par mole de fluorure de cyanuryle.

6. Procédé selon au moins l'une des revendications 1 à 3, caractérisé en ce que, lors de la réaction du résidu avec un mélange d'hydroxyde, d'hydrogénocarbonate et de carbonate de métaux alcalins, on utilise ces constituants, selon leurs proportions dans le mélange, en des quantités selon les revendications 4 et 5, par mole de fluorure de cyanuryle.

7. Procédé selon au moins l'une des revendications 1 à 6, caractérisé en ce qu'on ajoute les hydroxydes, hydrogénocarbonates ou carbonates de métaux alcalins ou leurs mélanges en l'état au résidu contenant du fluorure de cyanuryle.

8. Procédé selon au moins l'une des revendications 1 à 6, caractérisé en ce qu'on ajoute les hydroxydes, hydrogénocarbonates ou carbonates de métaux alcalins ou leurs mélanges au résidu contenant du fluorure de cyanuryle, sous forme d'une suspension dans un solvant aprotique, que l'on peut séparer par distillation du solvant servant de milieu réactionnel.

9. Procédé selon au moins l'une des revendications 1 à 6 et 8, caractérisé en ce qu'on ajoute les hydroxydes, hydrogénocarbonates ou carbonates de métaux alcalins ou leurs mélanges au résidu contenant du fluorure de cyanuryle, en suspension dans le solvant aprotique dipolaire servant de milieu réactionnel.

10. Procédé selon au moins l'une des revendications 1 à 6, caractérisé en ce qu'on ajoute le résidu contenant du fluorure de cyanuryle aux hydroxydes, hydrogénocarbonates ou carbonates de métaux alcalins, ou à leurs mélanges.
